# EUROPÄISCHE PATENTSCHRIFT

(11) **EP 3 178 376 B1**
(45) Veröffentlichungstag und Bekanntmachung des Hinweises auf die Patenterteilung: **11.07.2018**
(21) Anmeldenummer: 16202569.6
(22) Anmeldetag: 07.12.2016
(51) Int. Cl.: A61B 3/04, A61B 3/11

(54) **MESSBRILLE**
TRIAL FRAME
MONTURE D'ESSAI

(30) Priorität: 11.12.2015 DE 102015225017
(43) Veröffentlichungstag der Anmeldung: 14.06.2017
(73) Patentinhaber: Oculus Optikgeräte GmbH, 35582 Wetzlar (DE)
(72) Erfinder: FEIERTAG, Carsten, 35410 Hungen (DE)
(74) Vertreter: advotec.

(56) Entgegenhaltungen:
- DE-U1-202006 000 361
- DE-U1-202011 105 667
- GB-A- 208 028
- US-A- 2 256 491

## Beschreibung

Die Erfindung betrifft eine Messbrille zur Bestimmung der subjektiven Refraktion eines Probanden, mit zwei Glashaltevorrichtungen zur Aufnahme von Einsteckgläsern, einer Brücke, die die Glashaltevorrichtungen relativ zueinander in einem Abstand verstellbar verbindet, einer Nasenauflagevorrichtung, mit einer verschwenkbaren und höhenverstellbaren Nasenauflage, und zwei Bügeln, die längenveränderbar und höhenverstellbar ausgebildet sind, wobei die Brücke und die Glashaltevorrichtungen zusammen eine Führungseinrichtung zur Führung der bewegbaren Glashaltevorrichtungen in Längsrichtung der Brücke ausbilden, wobei die Brücke ein Spindelgetriebe mit einer Gewindespindel zur Abstandsverstellung der Glashaltevorrichtungen aufweist.

Derartige Messbrillen werden regelmäßig zur Bestimmung von Brillengläsern eingesetzt. Dabei werden die Messbrille einem Probanden bzw. einer Person angepasst und Einsteckgläser bzw. Probiergläser, die leicht austauschbar sind, in die Messbrille eingesetzt. Die Einsteckgläser sind drehbar angeordnet, um astigmatische Fehler in ihrer Achslage korrigieren zu können. Seiten- und Höhenfehler, die über Prismen korrigiert werden, müssen ebenfalls einstellbar sein, weshalb die Messbrille genau auf einen Probanden bzw. Patienten angepasst werden muss. Beispielsweise muss ein Pupillenabstand eingestellt werden können, wobei eine Pupillenmitte eines jeden Auges separat einstellbar sein soll. Eine Höhenanpassung der Messbrille erfolgt über die Nasenauflage, die auch schwenkbar an der Brücke angeordnet ist. Über ein Verschwenken der Nasenauflage und eine Längeneinstellung der Bügel kann ein Abstand der Messbrille relativ zu den Augen eingestellt werden. Zur Anpassung an unterschiedliche Ohrformen sind die Bügel in der Höhe und seitlich schwenkbar ausgebildet. Eine derartige Messbrille ist unter anderem aus der EP 0 567 817 B1 bekannt.

DE 20 2011 105667 offenbart eine Messbrille mit einer Nasenauflage, zwei verstellbaren Bügeln, zwei Glashaltern und einer Brücke, welche eine Gewindespindel und eine Führungsnut umfasst. Die Gewindespindel ist in eine Gewindebuchse des Glashalters eingeschraubt. Da eine Anpassung der Messbrille immer unmittelbar am Kopf des Probanden durch die zu untersuchende Person erfolgt, müssen die Bedienelemente der Messbrille stets leicht bedienbar und ergonomisch handhabbar angeordnet sein. Gleichwohl sollte der Proband bei der Anpassung der Messbrille nicht unnötig durch die Bedienung der Messbrille belästigt werden. So sind beispielsweise ein Verklemmen von Haaren des Probanden an der Messbrille oder Handbewegungen innerhalb des näheren Gesichtsfeldes nicht erwünscht.

Aufgrund der vielfältigen Verstellmöglichkeiten sind die bekannten Messbrillen aus einer Vielzahl von Einzelteilen ausgebildet, wodurch eine Herstellung der Messbrille aufwendig ist. So ist zur Höhenverstellung der Nasenauflage regelmäßig ein Verstellgetriebe erforderlich, wobei das Verstellgetriebe zur Umwandlung einer rotatorischen Verstellbewegung in eine translatorische Bewegung der Nasenauflage bei einer gewünschten Getriebeübersetzung aus einer Vielzahl von Getriebeelementen bzw. Zahnrädern ausgebildet ist.

Bei den bekannten Messbrillen werden die Glashaltevorrichtungen zur Anpassung des Pupillenabstands mittels Gewindespindeln, die innerhalb der Brücke angeordnet sind, verschoben. An den Glashaltevorrichtungen sind dazu Spindelmuttern ausgebildet oder angeordnet. So ist es beispielsweise bekannt, eine Glashaltevorrichtung in einem Kunststoffspritzgussverfahren herzustellen und die Gewindemutter an der Glashaltevorrichtung anzuformen. Dabei wird eine Gewindespindel in einer Form umspritzt, wobei die Gewindespindel zum Entformen der Glashaltevorrichtung aus der Gewindemutter herausgeschraubt werden muss. Durch Abkühlung oder Schwindung des Kunststoffmaterials kann sich jedoch die Gewindemutter derart verformen, dass ein Gewinde für die Gewindespindel nachgeschnitten werden muss.

Auch wird bei einigen Messbrillen die Brücke aus einem Metallrohr hergestellt, in das eine Nut gefräst wird. Innenflanken der Nut dienen dabei als eine Längsführung für die Glashaltevorrichtungen, sodass die Glashaltevorrichtungen transversal verschoben werden können. Die so ausgebildete Führungseinrichtung muss eine vergleichsweise genaue Passung aufweisen, um ein sagittales Verkippen der Glashaltevorrichtungen soweit als möglich auszuschließen. Die Brücke muss nach einem Fräsen der Nut daher regelmäßig nachgearbeitet werden, um die gewünschte Spielpassung auszubilden. Auch diese erforderlichen Fertigungsschritte sind relativ zeitaufwendig.

Der vorliegenden Erfindung liegt daher die Aufgabe zugrunde, eine Messbrille vorzuschlagen, die qualitativ hochwertig und gleichzeitig kostengünstig herzustellen ist.

Diese Aufgabe wird durch eine Messbrille mit den Merkmalen des Anspruchs 1 gelöst.

Die erfindungsgemäße Messbrille zur Bestimmung der subjektiven Refraktion eines Probanden umfasst zwei Glashaltevorrichtungen zur Aufnahme von Einsteckgläsern, eine Brücke, die die Glashaltevorrichtungen relativ zueinander in einem Abstand verstellbar verbindet, eine Nasenauflagevorrichtung, mit einer verschwenkbaren und höhenverstellbaren Nasenauflage, sowie zwei Bügel, die längenveränderbar und höhenverstellbar ausgebildet sind, wobei die Brücke und die Glashaltevorrichtungen zusammen eine Führungseinrichtung zur Führung der bewegbaren Glashaltevorrichtungen in Längsrichtung der Brücke ausbilden, wobei die Brücke ein Spindelgetriebe mit einer Gewindespindel zur Abstandsverstellung der Glashaltevorrichtungen aufweist, wobei an den Glashaltevorrichtungen jeweils zumindest ein Führungsvorsprung ausgebildet ist, der in eine in der Brücke ausgebildeten Führungsnut eingreift, wobei an den Glashaltevorrichtungen jeweils offene Gewindeabschnitte ausgebildet sind, wobei die Gewindespindel mit dem Gewindeabschnitt in Eingriff steht.

Die nachfolgend angegebenen Lage- und Richtungsbezeichnungen beziehen sich immer auf den Körper oder die Körperebenen eines Probanden bzw. Patienten dem die Messbrille angepasst ist.

Demnach ist die Führungseinrichtung so ausgebildet, dass die Glashaltevorrichtungen in Längsrichtung der Brücke, das heißt transversal bewegbar sind. So wird es möglich, einen Relativabstand der Glashaltevorrichtungen zur Anpassung der Messbrille an einen Pupillenabstand einzustellen. Diese Abstandsverstellung erfolgt mit dem Spindelgetriebe, welches zumindest eine Gewindespindel aufweist, und selbsthemmend ausgebildet sein kann. Für beide Glashaltevorrichtungen kann eine einzelne Gewindespindel mit gegenläufigen Gewindegängen in Gewindeabschnitten oder jeweils für eine Glashaltevorrichtung eine eigene Gewindespindel vorhanden sein. Die Gewindespindel greift dabei in einen Gewindeabschnitt der Glashaltevorrichtung ein, wobei der Gewindeabschnitt offen ausgebildet ist, das heißt an der Glashaltevorrichtung ist der Gewindeabschnitt in Art einer Zahnstange bzw. mit einem Kreissektor einer Gewindebohrung ausgebildet. Der Gewindeabschnitt kann dann integral mit der Glashaltevorrichtung, beispielsweise durch Spritzgießen einstückig ausgebildet werden. Mittels einer Drehbewegung der Gewindespindel kann eine translatorische Bewegung der Glashaltevorrichtung entlang einer Längsachse der Brücke bewirkt werden. Ein umständliches Entformen der Glashaltevorrichtung oder ein gegebenenfalls erforderliches Nachschneiden des Gewindeabschnitts ist nicht mehr erforderlich. Der Führungsvorsprung kann ebenso an der Glashaltevorrichtung angeformt sein. Der Führungsvorsprung ist vorzugsweise an einer Seitenfläche der Glashaltevorrichtung angeordnet, wobei an einer gegenüberliegenden Seitenfläche auch ein weiterer Führungsvorsprung oder auch mehrere Führungsvorsprünge an einer Seitenfläche angeordnet sein können. Wesentlich ist, dass der Führungsvorsprung in die Führungsnut eingreift, die in der Brücke ausgebildet ist. Neben Führungsflächen der Führungseinrichtung, die an Seitenflächen der Glashaltevorrichtung anliegen, werden durch den Führungsvorsprung und die Führungsnut weitere Führungsflächen geschaffen, die eine weitgehend spielfreie Führung der Glashaltevorrichtungen in der Brücke gewährleisten. Ein radiales Verkippen der Glashaltevorrichtungen relativ zur Gewindespindel, wie aus dem Stand der Technik bekannt, kann so wirkungsvoll verhindert werden.

Die Brücke kann einen Längsschlitz aufweisen, der parallele Führungsflächen für die Glashaltevorrichtungen ausbildet, wobei die Führungsnut an bzw. in zumindest einer der Führungsflächen ausgebildet sein kann. Die Führungsflächen des Längsschlitzes können dann an Seitenflächen der Glashaltevorrichtungen anliegen. In einer der Führungsflächen oder auch in beiden Führungsflächen kann jeweils ein Längsschlitz ausgebildet sein, wobei der Längsschlitz übereinstimmend mit dem Führungsvorsprung bzw. Führungsvorsprüngen ausgebildet sein kann. Die Führungsnut kann in Art einer rechteckigen, spitz zulaufenden oder halbrunden Nut ausgebildet sein. Die Führungsnut verhindert folglich auch ein Herausfallen der Glashaltevorrichtungen aus dem Längsschlitz. Ein sagittales Verkippen der Glashaltevorrichtungen kann insbesondere durch die zwischen der Führungsnut und dem Führungsvorsprung ausgebildeten horizontalen Führungsflächen der Führungseinrichtung wirksam verhindert werden. Eine besondere Passgenauigkeit des Längsschlitzes ist dann nicht mehr zwingend erforderlich.

Insbesondere kann in Längsrichtung der Brücke eine Durchgangsbohrung zur Aufnahme der Gewindespindel ausgebildet sein, wobei die Durchgangsbohrung dann an den Längsschlitz anschließt. Der Längsschlitz ragt demnach in die Durchgangsbohrung hinein, wobei ein Durchmesser der Durchgangsbohrung genauso groß oder größer als eine Breite des Längsschlitzes sein kann. Auch wird eine Montage der Messbrille wesentlich vereinfacht, dadurch, dass die Gewindespindel zusammen mit der Glashaltevorrichtung in die Durchgangsbohrung mit dem Längsschlitz eingeschoben werden muss.

Besonders vorteilhaft ist es, wenn an der Glashaltevorrichtung eine Mehrzahl von stegförmigen Führungsvorsprüngen ausgebildet ist. Die stegförmigen Führungsvorsprünge können an beiden Seitenflächen der Glashaltevorrichtung angeordnet sein. Dabei können an einer Seitenfläche beispielsweise zwei voneinander beabstandete stegförmige Führungsvorsprünge und an einer gegenüberliegenden Seitenfläche ein einzelner stegförmiger Führungsvorsprung angeordnet bzw. ausgebildet sein. Die sich hieraus ergebende Auflage der Glashaltevorrichtung an drei Abstützpunkten der Führungseinrichtung verhindert ein Verkanten bzw. Verklemmen der Glashaltevorrichtung in der Führungsnut.

Die Messbrille wird besonders kostengünstig herstellbar, wenn die Brücke aus einem Strangpressprofil ausgebildet ist. Das Strangpressprofil kann vorteilhaft bereits den Querschnitt der Brücke aufweisen, sodass keine oder nur noch unwesentliche Nacharbeiten an dem Strangpressprofil erforderlich sind. Da dann gegebenenfalls auch an dem Strangpressprofil keine Fräsarbeiten durchgeführt werden müssen, wird das Problem eines Aufbiegens des Längsschlitzes vermieden. Darüber hinaus ist ein Strangpressprofil, beispielsweise aus Aluminium oder Titan, kostengünstig erhältlich.

Die Gewindespindel kann an einem Innenlager radial und an einem Außenlager radial und axial gelagert sein. Das Innenlager kann daher als ein Loslager ausgebildet sein und das Außenlager als ein Festlager der Gewindespindel. Das Innenlager kann eine Kunststoffbuchse sein, die lediglich in eine Bohrung in der Brücke zur Aufnahme der Gewindespindel eingesetzt wird. Das Außenlager kann aus Kunststoff ausgebildet sein, wobei das Außenlager an einem äußeren, transversalen Ende der Brücke an dieser fest fixiert sein kann. Diese Lagerung der Gewindespindel wird unter anderem dadurch erforderlich, dass die Glashaltevorrichtung kein geschlossenes Gewinde aufweist. Wenn es sich bei dem Innenlager lediglich um eine Kunststoffbuchse handelt, sind deren Mehrkosten alleine schon durch das vereinfachte Entformen der Glashaltevorrichtung bei weitem aufgewogen.

Das Außenlager kann formschlüssig in die Führungsnut eingreifen. Dadurch wird es möglich, eine radiale Drehung des Außenlagers zu verhindern und das Außenlager auch, beispielsweise durch eine Klemmpassung in der Führungsnut, gegen axiale Bewegungen zu sichern. Optional kann auch vorgesehen sein, das Außenlager mit einer Schraubverbindung an der Brücke zu fixieren. Das Außenlager kann mittels Kunststoffspritzguss hergestellt werden.

Bedienelemente der Messbrille können besonders gut betätigt werden, wenn diese zylinderförmig oder kegelstumpfförmig und aus flexiblem Kunststoffmaterial einstückig ausgebildet sind. Insbesondere beim Erfassen eines kegelstumpfförmigen Bedienelements durch Pronation von 2 bis 3 Fingern kann das Bedienelement am größten Durchmesser des Kegelstumpfs, wenn eine Feineinstellung erfolgt, oder wahlweise an der Umfangsfläche des Kegelstumpfs, wenn eine Schnellverstellung erfolgt, ergriffen werden. Darüber hinaus ist das Bedienelement durch das flexible Kunststoffmaterial haptisch angenehm erfassbar und verhindert ein Durchrutschen des Bedienelements zwischen Fingern. Durch die einstückige Ausbildung ist das Bedienelement kostengünstig herzustellen und verringert eine Teileanzahl der Messbrille. Zur Verbindung mit Betätigungswellen oder Spindeln der Messbrille kann das Bedienelement eine Ausnehmung zum formschlüssigen Aufstecken mit einem Rastvorsprung oder einer Rastausnehmung aufweisen.

Die Brücke kann aus zwei Brückenabschnitten ausgebildet sein, die mittels einer Schwenkachse fest verbunden sein können, wobei an der Schwenkachse die Nasenauflagevorrichtung schwenkbar gelagert sein kann. In einer besonders einfachen Ausführungsform können dann die Brückenabschnitte lediglich mittels eines Stifts, der in die jeweiligen Brückenabschnitte eingesteckt ist und die Schwenkachse ausbildet, verbunden sein. Die Brückenabschnitte sind dann auch in ihrer äußeren Gestalt unabhängig von der Schwenkachse bzw. der Verbindung mit der Nasenauflagevorrichtung ausbildbar. Die Brückenabschnitte können einfach mittels einer Schraube oder auch durch Verkleben gegen Verdrehen oder Herausziehen der Schwenkachse an dieser gesichert werden. Besonders vorteilhaft ist es dann auch, wenn ausschließlich eine axiale Klemmung der Nasenauflagevorrichtung an der Schwenkachse erfolgt.

Die Nasenauflagevorrichtung kann ein Verstellgetriebe zur Höhenverstellung der Nasenauflage aufweisen, welches selbsthemmend ausgebildet sein kann, wobei das Verstellgetriebe eine Zahnstange und ein Kegelschneckenrad, die miteinander in Eingriff stehen, aufweisen kann. Dadurch, dass das Verstellgetriebe zur Höhenverstellung der Nasenauflage eine Zahnstange aufweisen kann, kann die Zahnstange translatorisch bewegt und so eine Höhenverstellung der Nasenauflage bewirkt werden. Weiter ist es dann durch die Zahnstange leicht möglich, das Verstellgetriebe selbsthemmend auszubilden. Die Zahnstange steht dann mit dem Kegelschneckenrad in Eingriff, sodass eine Drehung des Kegelschneckenrades die translatorische Bewegung der Zahnstange bewirken kann. Dadurch, dass das Kegelschneckenrad bezogen auf eine Längsachse des Kegelschneckenrades einen kegelförmigen Außendurchmesser mit einer Verzahnung ausbilden kann, kann das Kegelschneckenrad relativ zur Zahnstange so angeordnet werden, dass die Längsachse des Kegelschneckenrades in einem Winkel β, welcher einem halben Öffnungswinkel α des Kegelschneckenrades entspricht, angeordnet werden. Bei einer longitudinalen Höhenverstellung der Zahnstange bzw. einer in einer Sagittalebene relativ zur Brücke oder Glashaltevorrichtung höhenverstellbaren Nasenauflage ist das Kegelschneckenrad vorzugsweise dann so angeordnet, dass sich die Längsachse in einer distalen Richtung erstreckt. Dadurch wird es dann möglich, die Höhenverstellung der Nasenauflage vorzunehmen, ohne dass eine Hand einer Bedienperson in das unmittelbare Gesichtsfeld eines Probanden bzw. Patienten gelangt. Darüber hinaus wird dann ein Einsetzen von Einsteckgläsern in die Glashaltevorrichtungen nicht durch ein Bedienelement des Verstellgetriebes unmittelbar im Bereich der Nase des Probanden behindert. Die Messbrille ist dann so einfacher handhabbar und wird von dem Probanden weniger als störend empfunden.

Das Verstellgetriebe kann von der Zahnstange und dem Kegelschneckenrad ausgebildet sein. Das Verstellgetriebe ist dann alleine aus zwei bewegbaren Getriebekomponenten ausgebildet. Eine Montage des Verstellgetriebes wird dadurch wesentlich vereinfacht, wodurch eine kostengünstige Herstellung der Messbrille möglich wird. Eine Verzahnung des Kegelschneckenrades kann dann in Art eines kegeligen Gewindes verlaufen. Da eine Verstellung der Nasenauflage im Handbetrieb erfolgt, werden nur geringe Kräfte über das Verstellgetriebe übertragen, weshalb für die Ausbildung von Zähnen bzw. einer Zahngeometrie der Zahnstange und des Kegelschneckenrades vereinfachte Geometrien verwendet werden können.

Besonders vorteilhaft ist es, wenn das Kegelschneckenrad einen Öffnungswinkel α von 60° bis 120°, bevorzugt von 90° aufweist. Wenn dann das Kegelschneckenrad mit der Zahnstange in Eingriff steht, ist das Kegelschneckenrad relativ zur Zahnstange um einen Winkel β von 30° bis 60°, bevorzugt von 45° geneigt. Da die Zahnstange im Wesentlichen in einer longitudinalen Richtung verstellt wird, ist es weiter vorteilhaft, wenn das Kegelschneckenrad in einer distalen Richtung bzw. nach oben hin geneigt an der Zahnstange angeordnet ist, sodass ein mit dem Kegelschneckenrad in Verbindung stehendes Bedienelement oberhalb eines Nasenrückens eines Probanden angeordnet sein kann. Bei einer Verstellung der Nasenauflage durch eine Bedienperson befindet sich dann eine betätigende Hand der Bedienperson nicht unmittelbar vor den Augen bzw. im näheren Gesichtsfeld des Probanden. Darüber hinaus ist das Bedienelement dann für die Bedienperson ergonomisch vorteilhaft angeordnet und stört nicht bei einem Wechsel der Einsteckgläser.

Weiter kann das Kegelschneckenrad eine Steigung von 1 mm bis 3 mm, bevorzugt von 2 mm aufweisen. Dann kann die Nasenauflage mit einer einzigen Umdrehung eines Bedienelementes der Nasenauflagevorrichtung in einer longitudinalen Richtung um die entsprechende Länge verstellt werden.

Das Kegelschneckenrad kann an eine Betätigungswelle angeformt sein, wobei die Betätigungswelle dann so ausgebildet sein kann, dass auf die Betätigungswelle ein Bedienelement unmittelbar aufsteckbar ist. Beispielsweise kann das Kegelschneckenrad einstückig in einem Spritzgussverfahren hergestellt werden. Das Kegelschneckenrad kann daher aus Kunststoff oder Metall bestehen. Insgesamt kann so eine Teileanzahl der Messbrille weiter reduziert werden. Ein distales Ende der Betätigungswelle kann so ausgebildet sein, dass das Bedienelement formschlüssig mit dem distalen Ende verbindbar ist. Beispielsweise kann an dem distalen Ende eine kreissegmentförmige Materialausnehmung ausgebildet sein, an die das Bedienelement angepasst ist. Weiter kann an dem distalen Ende eine Rastausnehmung oder ein Rastvorsprung für eine Rastverbindung mit dem Bedienelement ausgebildet sein.

So kann dann auch das Bedienelement oberhalb der Brücke angeordnet sein, wodurch das Bedienelement dann an einem Rand bzw. außerhalb eines Gesichtsfeldes eines Probanden angeordnet ist.

Die Nasenauflagevorrichtung kann an einer Schwenkachse der Brücke mittels einer radialen Klemmung gelagert sein. Eine radiale Klemmung ist besonders einfach herstellbar und kann beispielsweise einen die Schwenkachse umgebenden Ring mit einem Schlitz oder einer Feder aufweisen. Die radiale Klemmung kann auch in ihrer Klemmkraft einstellbar ausgebildet sein. Wesentlich ist, dass eine selbsthemmende Klemmung der Nasenauflagevorrichtung an der Schwenkachse erfolgt.

Die Glashaltevorrichtung kann einen drehbaren Ring aufweisen, an dem Glasaufnahmen ausgebildet sind, wobei eine Betätigungswelle eines Rings eine einstellbare Bremseinrichtung aufweisen kann, wobei die Bremseinrichtung ein exzentrischer Kunststoffring sein kann. Der drehbare Ring mit den Glasaufnahmen kann aus Kunststoff oder Leichtmetall hergestellt werden, wobei die Betätigungswelle mit einem Zahnrad an einer an dem drehbaren Ring ausgebildeten Verzahnung eingreifen kann, sodass mittels einer Drehung der Betätigungswelle der drehbare Ring drehbar ist. Die Betätigungswelle ist dann in Richtung eines Mittelpunkts des Rings verlaufend angeordnet. Um ein unbeabsichtigtes Verdrehen des Rings zu verhindern, kann der Kunststoffring die Betätigungswelle umgeben, sodass ein Drehwiderstand der Betätigungswelle ausbildbar ist. Wenn der Kunststoffring exzentrisch ausgebildet ist, kann durch eine radiale Drehung des Kunststoffrings auf der Betätigungswelle ein größerer oder ein kleinerer Drehwiderstand nach Bedarf eingestellt werden.

Die Glashaltevorrichtungen können jeweils vier vordere und zwei hintere Glasaufnahmen aufweisen. Durch die Vielzahl der vorderen bzw. ventralen und hinteren bzw. dorsalen Glasaufnahmen wird die Messbrille besonders universell einsetzbar. So können gleichzeitig eine Reihe von Einsteckgläsern, beispielsweise zur Korrektur von Kurzsichtigkeit, Weitsichtigkeit und Astigmatismus eingesetzt werden. Die Glasaufnahmen können jeweils aus zumindest zwei oder drei Stegen ausgebildet sein, die Einsteckgläser an deren Umfang haltern können.

Die Glasaufnahmen der Glashaltevorrichtungen können jeweils zumindest einen Andruckfinger zum Klemmen eines Einsteckglases aufweisen, wobei die jeweiligen Andruckfinger in einer Reihenanordnung an der Glashaltevorrichtung angeordnet und über eine gemeinsame Blattfeder mit einer Andruckkraft beaufschlagbar sind. Wenn die Glasaufnahmen stegförmig ausgebildet sind, kann beispielsweise an einem der Stege für jeweils ein Einsteckglas ein Andruckfinger ausgebildet oder angeordnet sein. Aus dem Stand der Technik ist es bekannt, diese Andruckfinger mittels jeweils einzelner Schenkelfedern aus Federdraht mit der Andruckkraft zu beaufschlagen. Um eine Teileanzahl der Messbrille zu reduzieren und eine Montage zu vereinfachen ist es daher vorteilhaft, eine einzelne Blattfeder anstelle einer Mehrzahl von Schenkelfedern einzusetzen. Dies setzt voraus, dass die Andruckfinger stets benachbart und in einer Reihenanordnung an der Glashaltevorrichtung angeordnet sind. Die Blattfeder kann auch so ausgebildet sein, dass die Blattfeder mehrere fingerförmige Blattfederabschnitte ausbildet, die jeweils alleine auf einen Andruckfinger wirken.

Bügelenden der Bügel können jeweils flexibel ausgebildet sein, wobei die Bügelenden jeweils auch schlaufenförmig ausgebildet sein können. Die dorsalen Bügelenden der Bügel können vorteilhaft eine sichel- oder halbmondförmige Schlaufe ausbilden, die eine Ohrmuschel bzw. eine Ohrwulst hintergreifen kann, um so ein Herabfallen der Messbrille vom Kopf eines Probanden zu verhindern. Durch die schlaufenförmige Ausbildung der Bügelenden werden diese flexibel und können sich besonders gut an eine Ohrmuschel anpassen, sodass für einen Probanden gegebenenfalls unangenehme Druckstellen an der Ohrmuschel vermieden werden. Die flexible Ausbildung der Bügelenden wird vereinfacht, wenn die Bügelenden aus einem zumindest teilweise flexibel formbaren Kunststoffmaterial bestehen.

Bügelenden der Bügel können jeweils einstückig sowie aus einem weichen und einem vergleichsweise harten Kunststoffmaterial ausgebildet sein. Dabei kann vorgesehen sein, die dorsalen Bügelenden aus dem weichen Kunststoffmaterial auszubilden und den Bügel selbst abschnittsweise aus dem harten Kunststoffmaterial auszubilden. Der Bügel ist dann mittels Kunststoffspritzguss kostengünstig und einfach herstellbar. Das weiche Kunststoffmaterial kann flexibel sein, sodass Druckstellen an einer Ohrmuschel eines Probanden vermieden werden. Zumindest ein an das dorsale Bügelende anschließender gerader Bügelabschnitt des Bügels kann dann aus dem vergleichsweise harten Kunststoffmaterial ausgebildet sein, da dieser Bügelabschnitt auch vergleichsweise steif sein muss. Der Bügelabschnitt kann auch zu einem ventralen, vorderen Bügelende hin in einen metallenen Bügelabschnitt übergehen. Beide Bügelabschnitte können teleskopartig ineinanderschiebbar ausgebildet sein.

Ein vorderes bzw. ventrales Ende des Bügels kann gekröpft ausgebildet sein. Dadurch wird es möglich, den Bügel möglichst tief, also in proximaler Richtung an den Glashaltevorrichtungen anzuordnen. Eine Bedienperson kann dann die Augen auch ungehindert von der Seite her betrachten und Einsteckgläser in hintere Glasaufnahmen einsetzen. Auch kann dann einfacher ein Abstand der Messbrille zu den Augen eingestellt bzw. von der Bedienperson gemessen werden. Dadurch, dass der Bügel bzw. dessen ventrales Ende gekröpft ist, muss der Bügel gegenüber einer horizontalen Transversalebene nicht wesentlich geneigt sein, sodass der Bügel wie gewohnt an eine Ohrmuschel anpassbar ist.

Die Messbrille kann zwei Polarisationsfiltervorrichtungen umfassen. Die Messbrille kann dann auch mit Polarisationsfiltervorrichtungen eingesetzt werden.

Die Polarisationsfiltervorrichtungen können jeweils an den Glashaltevorrichtungen mittels einer Rastverbindung lösbar befestigbar sein. Da die Polarisationsfiltervorrichtungen für optionale Sehtests mit der Messbrille nutzbar sein sollen, können diese unabhängig von den Glashaltevorrichtungen an der Messbrille lösbar befestigt werden. Dadurch, dass diese lösbare Befestigung mit einer Rastverbindung an der Glashaltevorrichtung erfolgt, kann die Polarisationsfiltervorrichtung besonders schnell und einfach adaptiert und auch wieder entfernt werden. Die Rastverbindung kann auch so ausgebildet sein, dass eine Klemmkraft bewirkt wird, die eine unerwünschte Bewegung der Polarisationsfiltervorrichtung an der Glashaltevorrichtung verhindert. Die Rastverbindung kann dann als spielfreie Verbindung ausgebildet sein, sodass die Polarisationsfiltervorrichtung besonders präzise bedient werden kann.

An einem Befestigungsende eines Halters der Polarisationsfiltervorrichtung kann eine einstückige Federklemme ausgebildet sein, die einen an der Glashaltevorrichtung angeformten Steg hintergreifen kann, wobei an der Federklemme bzw. dem Befestigungsende ein Betätigungshebel angeformt sein kann, mittels dem die Federklemme offenbar ist. Das Befestigungsende kann auch einstückig und vollständig aus Kunststoff ausgebildet sein und die Federklemme umfassen, um eine Teileanzahl der Messbrille noch weiter zu reduzieren. Das Befestigungsende kann auch in Form einer Kralle ausgebildet sein, die den Steg hintergreift, derart, dass der Halter an den Steg eingehängt werden kann. Weiter kann an dem Steg oder der Federklemme eine Rastnase oder Rastausnehmung angeformt sein, in die jeweils eine übereinstimmend ausgebildete Rastausnehmung bzw. Rastnase eingreifen kann. Wenn die Kralle an einer Kante des Stegs an diesen angreift bzw. an dieser Kante des Stegs eingehängt ist, kann die Federklemme an einer gegenüberliegenden Kante des Stegs angreifen und mit diesem verrasten. Um ein Lösen des Halters bzw. der Federklemme von der Glashaltevorrichtung bzw. dem Steg zu erleichtern kann an der Federklemme bzw. dem Befestigungsende der Betätigungshebel angeformt sein. Der Betätigungshebel kann so angeordnet sein, dass bei einer Betätigung des Betätigungshebels die Federklemme aufgebogen wird bzw. die Rastverbindung aufgelöst wird.

Die Polarisationsfiltervorrichtungen können einen zirkularen oder einen linearen Polarisationsfilter aufweisen, wobei die Polarisationsfiltervorrichtungen mit dem zirkularen oder linearen Polarisationsfilter jeweils eine Schwenkeinrichtung zum Ein- und Ausschwenken des zirkularen oder linearen Polarisationsfilters vor die Glashaltevorrichtung aufweisen können, wobei die Polarisationsfiltervorrichtungen mit dem linearen Polarisationsfilter jeweils eine Dreheinrichtung zum Drehen des linearen Polarisationsfilters vor der Glashaltevorrichtung aufweisen können.

Je nach Polarisation verwendeter Sehzeichen kann der zirkulare oder der lineare Polarisationsfilter ausgewählt werden. Der zirkulare und der lineare Polarisationsfilter kann jeweils mittels der Schwenkeinrichtung vor die Glashaltevorrichtung in das Gesichtsfeld eines Probanden eingeschwenkt oder ausgeschwenkt werden, um den entsprechenden Sehtest durchführen zu können. Wenn die Polarisationsfiltervorrichtungen über den linearen Polarisationsfilter verfügt, ist weiter die Dreheinrichtung zum Drehen des linearen Polarisationsfilters vor der Glashaltevorrichtung bzw. dem Gesichtsfeld des Probanden vorgesehen. Die Schwenkeinrichtung kann mit einem Scharnier ausgebildet sein, welches ein radiales oder axiales Verschwenken des Polarisationsfilters relativ zu einer Sehachse des Probanden ermöglicht. Die Dreheinrichtung kann mit einer Achse ausgebildet sein, welche ein radiales oder axiales Verdrehen des linearen Polarisationsfilters relativ zu einer Sehachse des Probanden ermöglicht. Dadurch wird es möglich, eine Orientierung bzw. Richtung einer Polarisation zu verändern. Die Polarisationsfiltervorrichtungen sind je nach Polarisation der Sehzeichen so flexibel einsetzbar.

Nachfolgend wird eine bevorzugte Ausführungsform der Messbrille anhand der Zeichnungen näher erläutert.

Es zeigen:
- **Fig. 1**: Eine perspektivische Ansicht einer Messbrille;
- **Fig. 2**: die Messbrille mit adaptierten Polarisationsfiltervorrichtungen;
- **Fig. 3**: eine Vorderansicht der Messbrille;
- **Fig. 4**: eine Rückansicht der Messbrille;
- **Fig. 5**: eine Seitenansicht der Messbrille;
- **Fig. 6**: eine Seitenansicht der Messbrille mit adaptierten Polarisationsfiltervorrichtungen;
- **Fig. 7**: eine Draufsicht der Messbrille;
- **Fig. 8**: eine perspektivische Detaildarstellung einer Glashaltevorrichtung mit einer Gewindespindel;
- **Fig. 9**: eine Teildarstellung einer Glashaltevorrichtung an einer Brücke;
- **Fig. 10**: eine Schnittansicht entlang einer Linie X-X aus **Fig. 9****;**
- **Fig. 11**: eine Teildarstellung der Glashaltevorrichtung und der Brücke aus **Fig. 9** in einer Seitenansicht;
- **Fig. 12**: eine Schnittdarstellung entlang einer Linie XII-XII aus **Fig. 11****;**
- **Fig. 13**: eine Unteransicht einer Nasenauflagevorrichtung;
- **Fig. 14**: eine Seitenansicht der Nasenauflagevorrichtung;
- **Fig. 15**: eine Rückansicht der Nasenauflagevorrichtung;
- **Fig. 16**: eine Querschnittansicht der Nasenauflagevorrichtung.

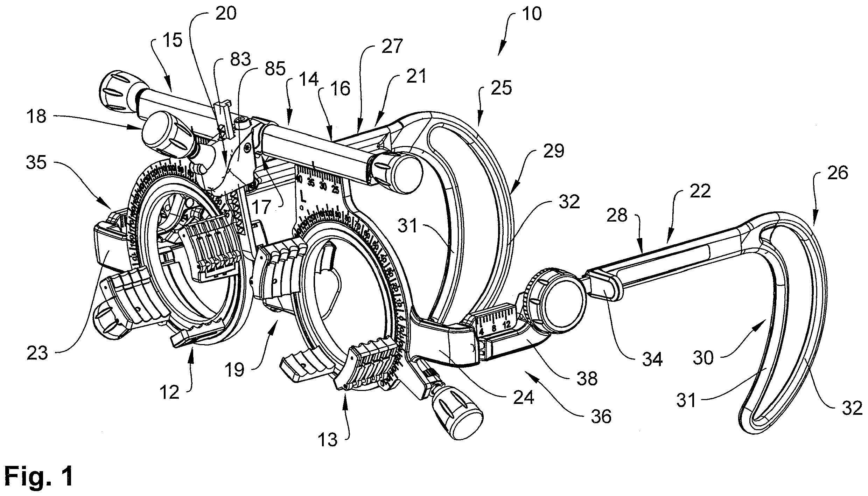

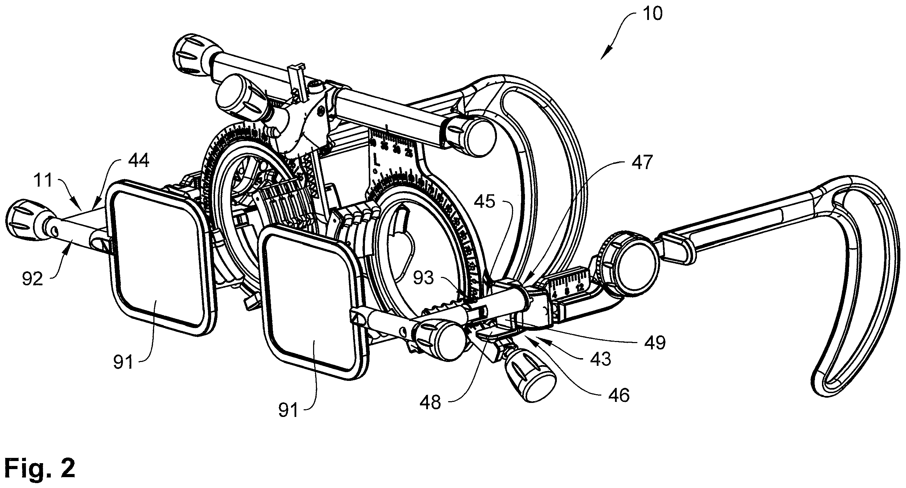

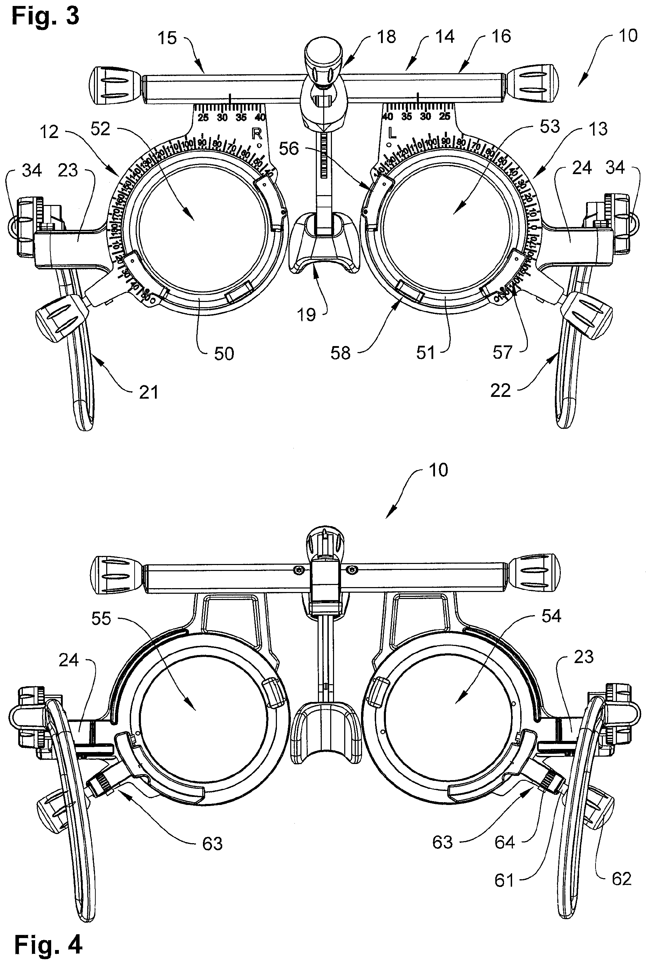

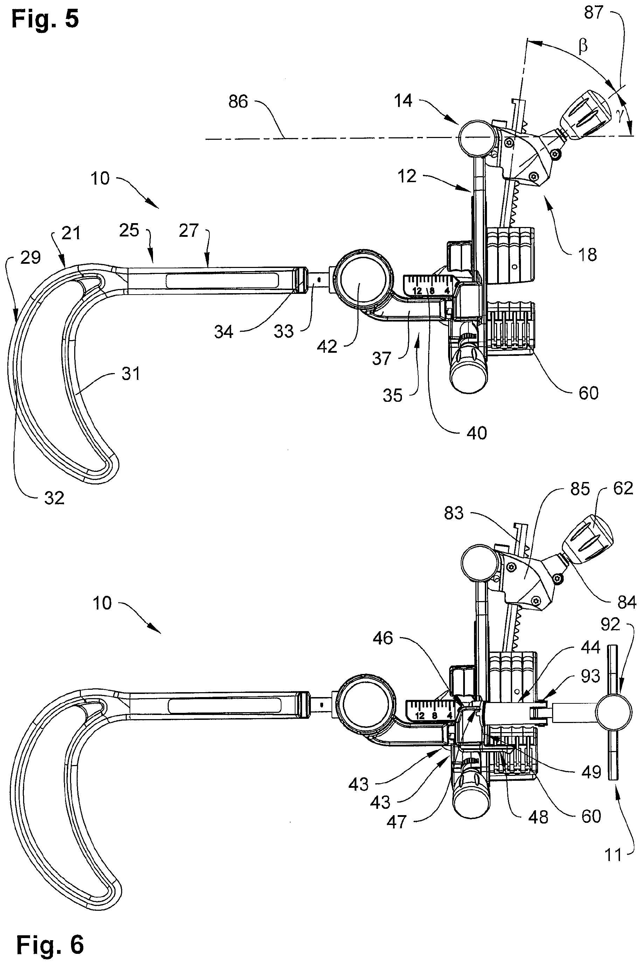

Eine Zusammenschau der **Fig. 1** bis **7** zeigt eine Messbrille 10 in verschiedenen Ansichten. Bei den Darstellungen in den **Fig. 2** und **6** sind Polarisationsfiltervorrichtungen 11 der Messbrille 10 an dieser adaptiert. Die Messbrille 10 dient zur Bestimmung der subjektiven Refraktion eines hier nicht dargestellten Probanden bzw. Patienten. Nachfolgende Richtungs- und Lagebezeichnungen beziehen sich daher stets auf Körperebenen eines Probanden mit angepasster Messbrille 10. Die Messbrille 10 umfasst zwei Glashaltevorrichtungen 12 und 13 zur Aufnahme von hier nicht näher dargestellten Einsteckgläsern sowie eine Brücke 14, die die Glashaltevorrichtungen 12 und 13 relativ zueinander in einem Abstand in transversaler Richtung einzeln verstellbar verbindet. Die Brücke ist aus zwei Brückenabschnitten 15 und 16, die mittels einer Schwenkachse 17 miteinander fest verbunden sind, ausgebildet. Die Glashaltevorrichtung 12 ist demnach an den Brückenabschnitt 15 und die Glashaltevorrichtung 13 an dem Brückenabschnitt 16 verstellbar befestigt. Weiter umfasst die Messbrille 10 eine Nasenauflagevorrichtung 18 mit einer Nasenauflage 19. Die Nasenauflagevorrichtung 18 bzw. die Nasenauflage 19 ist an der Schwenkachse 17 in einer Sagittalebene relativ zur Brücke 14 oder Glashaltevorrichtung 12 und 13 radial schwenkbar gelagert. Die Nasenauflage 19 ist weiter in longitudinaler Richtung relativ zur Brücke 14 oder den Glashaltevorrichtungen 12 und 13 höhenverstellbar. Die Höhenverstellung wird durch ein Verstellgetriebe 20 ermöglicht. Die Messbrille 10 umfasst weiter zwei Bügel 21 und 22, die an von den Glashaltevorrichtungen 12 und 13 jeweils ausgebildeten Stegen 23 bzw. 24 bewegbar angeordnet sind. Die Bügel 21 und 22 sind in sagittaler Richtung relativ zur Brücke 14 oder zu den Glashaltevorrichtungen 12 und 13 längenveränderbar und in longitudinaler Richtung höhenverstellbar ausgebildet.

Die Bügel 21 und 22 weisen jeweils flexibel ausgebildete Bügelenden 25 bzw. 26 auf, die zusammen mit jeweils einem Bügelabschnitt 27 bzw. 28 einstückig ausgebildet sind. Die Bügelenden 25 und 26 bestehen aus einem weichen Kunststoffmaterial und die Bügelabschnitte 27 bzw. 28 aus einem vergleichsweise harten Kunststoffmaterial. Die Bügelenden 25 und 26 werden zusammen mit den Bügelabschnitten 27 bzw. 28 in einem Spritzgussverfahren in einer gemeinsamen Form hergestellt. Die Bügelenden 25 und 26 bilden jeweils eine sichelförmige Schlaufe 29 bzw. 30 aus, wobei ventrale Schlaufenabschnitte 31 an einer Ohrmuschel bzw. Ohrwulst zur Anlage gelangen und dorsale Schlaufenabschnitte 32 eine elastische Verformung der Bügelenden 25 und 26 in Art einer Feder gewährleisten. Die Bügelenden 25 und 26 passen sich dadurch besonders gut an eine Ohrmuschel eines Probanden an. Die Bügelabschnitte 27 und 28 sind mit jeweils einem Führungsstab 33 längsbeweglich verbunden, sodass eine Längenverstellung der Bügel 21 und 22 möglich ist. Um die manuelle Längenverstellung zur erleichtern, sind an den Bügelabschnitten 27 und 28 jeweils Betätigungsvorsprünge 34 angeformt. Weiter sind ventrale Enden 35 und 36 der Bügel 21 bzw. 22 mit einem gekröpften Abschnitt 37 bzw. 38 ausgebildet. Die Abschnitte 37 und 38 sind jeweils an den Stegen 23 bzw. 24 an Achsen 39 schwenkbar befestigt, wobei an den Abschnitten 37 und 38 jeweils transluzente Maßstäbe 40 bzw. 41 befestigt sind. Die Maßstäbe 40 und 41 können zur Abstandsmessung relativ zu einem Auge bzw. dessen Apex genutzt werden. Die Abschnitte 37 und 38 sind weiter über Schwenkeinrichtungen 42 zur Verstellung einer Neigung der Bügelenden 25 bzw. 26 in longitudinaler Richtung mit den Führungsstäben 33 verbunden. Die Bügel 21 bzw. 22 können demnach in drei Richtungen, transversal, longitudinal und sagittal, verstellt und an einen Kopf eines Probanden angepasst werden.

Die Polarisationsfiltervorrichtungen 11 sind jeweils an den Glashaltevorrichtungen 12 und 13 mittels einer Rastverbindung 43 lösbar befestigbar. An Haltern 44 und 45 der Polarisationsfiltervorrichtungen 11 sind einstückige Federklemmen 46 ausgebildet, die über eine obere Haltenut 47 an den Stegen 23 bzw. 24 eingehängt werden können. Neben der Haltenut 47 weist die Federklemme 46 einen Betätigungshebel 48 auf, der eine hier nicht näher dargestellte Rastnase ausbildet. Diese Rastnase kann mit einer ebenfalls nicht dargestellten Rastnase bzw. Rastausnehmung unterhalb des Stegs 23 bzw. 24 verrasten, sodass die Federklemme 46 die Stege 23 und 24 hintergreift. Die aus Kunststoff ausgebildete, elastische Federklemme 46 ist von den Stegen 23 bzw. 24 dadurch lösbar, dass der Betätigungshebel 48 gedrückt wird. Dadurch wird ein Verbindungsabschnitt 49 der Federklemme 46, welcher die Haltenut 47 mit dem Betätigungshebel 48 verbindet, soweit aufgebogen, dass die Rastverbindung 43 gelöst und die Federklemme 46 von den Stegen 23 bzw. 24 entfernt werden kann. Insbesondere durch die Ausbildung der Rastverbindung 43 unter einer durch die Federklemme 46 bewirkten Federspannung wird es möglich, die Polarisationsfiltervorrichtungen 11 spielfrei und leicht handhabbar an den Stegen 23 und 24 zu adaptieren.

Die Polarisationsfiltervorrichtungen 11 weisen jeweils einen zirkularen oder einen linearen Polarisationsfilter 91 auf. Der hier gezeigte lineare Polarisationsfilter 91 ist mittels eines Scharniers 93 der Polarisationsfiltervorrichtung 11 vor den Glashaltevorrichtungen 12 bzw. 13 ein- und ausschwenkbar. Weiter ist der Polarisationsfilter vor den Glashaltevorrichtungen 12 bzw. 13 um eine Achse 92 der Polarisationsfiltervorrichtung 11 drehbar, derart, dass eine Richtung der Polarisation des linearen Polarisationsfilters 91 gewechselt werden kann.

Die Glashaltevorrichtungen 12 und 13 weisen jeweils einen drehbaren Ring 50 bzw. 51 auf, in dem vier vordere bzw. ventrale Glasaufnahmen 52 bzw. 53 angeordnet sind. Weiter sind an den Glashaltevorrichtungen 12 und 13 zwei hintere bzw. dorsale Glasaufnahmen 54 bzw. 55 angeordnet. Die vorderen Glasaufnahmen 52 und 53 sind aus Stegen 56, 57 und 58 ausgebildet, wobei die Stege 56 und 57 Andruckfinger 59 zum Klemmen von hier nicht dargestellten Einsteckgläsern aufweisen. Die Andruckfinger 59 werden über eine gemeinsame Blattfeder 60 mit einer Andruckkraft beaufschlagt.

Die Ringe 50 und 51 können über jeweils eine drehbare Betätigungswelle 61 mit einem Bedienelement 62 manuell gedreht und mit Hilfe einer Skalenteilung von 2,5° auf beispielsweise einen Winkel eines Zylinders eines Einsteckglases eingestellt werden. Um eine unbeabsichtigte Verstellung der Ringe 50 und 51 zu verhindern, ist an der Betätigungswelle 61 eine Bremseinrichtung 63 ausgebildet. Die Bremseinrichtung 63 ist aus einem exzentrischen Kunststoffring 64 ausgebildet, der die Betätigungswelle 61 umgibt und durch dessen radiale Drehung auf der Betätigungswelle 61 ein wahlweise größerer oder kleinerer Drehwiderstand der Betätigungswelle 61 eingestellt werden kann.

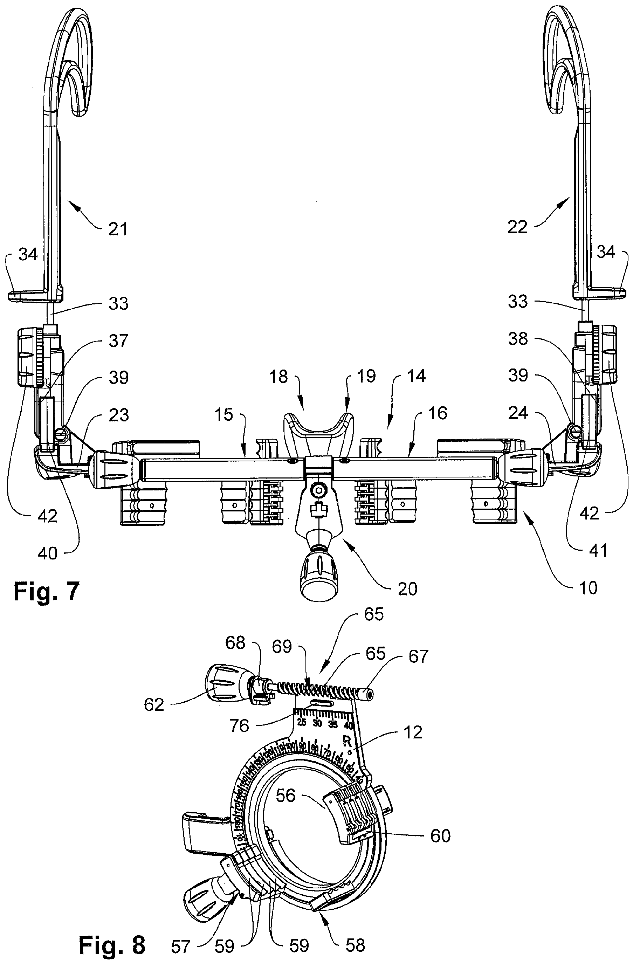

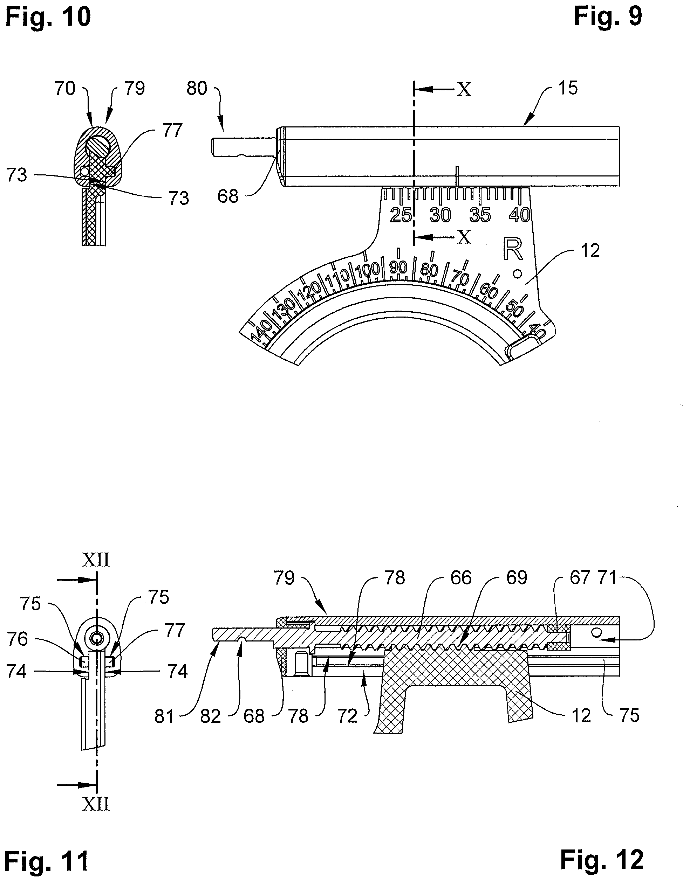

Eine Zusammenschau der **Fig. 8** bis **12** zeigt ein Spindelgetriebe 65 für jeweils die Glashaltevorrichtungen 12 und 13 mit einer Gewindespindel 66, einem Innenlager 67 und einem Außenlager 68 sowie einem Bedienelement 62. An den Glashaltevorrichtungen 12 und 13 ist jeweils ein Gewindeabschnitt 69 angeformt, mit dem die Gewindespindel 66 in Eingriff steht. Eine Drehung der Gewindespindel 66 bewirkt folglich eine Verstellung der Glashaltevorrichtung 12 bzw. 13 in transversaler Richtung. Die Brückenabschnitte 15 und 16 sind aus einem Strangpressprofil 70 aus Aluminium ausgebildet, wobei das Strangpressprofil 70 eine Durchgangsbohrung 71 aufweist, in die die Gewindespindel 66 mit dem Innenlager 67 eingesetzt ist. Die Gewindespindel 66 muss lediglich zusammen mit den Glashaltevorrichtungen 12 bzw. 13 und dem Außenlager 68 sowie dem Innenlager 67 in die Durchgangsbohrung 71 eingesteckt werden. Eine Sicherung des Außenlagers erfolgt mittels einer hier nicht näher dargestellten Schraube. Mit der Durchgangsbohrung 71 verbunden ist ein Längsschlitz 72, der parallele Führungsflächen 73 für Seitenflächen 74 der Glashaltevorrichtungen 12 bzw. 13 ausbildet. In den Führungsflächen 73 ist jeweils eine Führungsnut 75 ausgebildet. Die Glashaltevorrichtungen 12 und 13 bilden Führungsvorsprünge 76 und 77 aus, die in die Führungsnuten 75 eingreifen. Die Führungsnuten 75 bilden somit ihrerseits Führungsflächen 78 für die Führungsvorsprünge 76 und 77 aus. Dadurch wird eine besonders stabile und spielfreie Führung der Glashaltevorrichtungen 12 und 13 in einer so ausgebildeten Führungseinrichtung 79 gewährleistet, wobei die Führungseinrichtung 79 besonders einfach herstellbar ist.

Alle Bedienelemente 62 sind stets, wie hier am Beispiel der Gewindespindel 66 gezeigt, kegelstumpfförmig und aus flexiblem Kunststoffmaterial einstückig ausgebildet und auf ein Betätigungsende 80 lediglich aufgesteckt. Das Betätigungsende 80 weist zur Übertragung eines Drehmoments eine Passfläche 81 und zur Sicherung des Bedienelements 62 eine Rastausnehmung 82 auf.

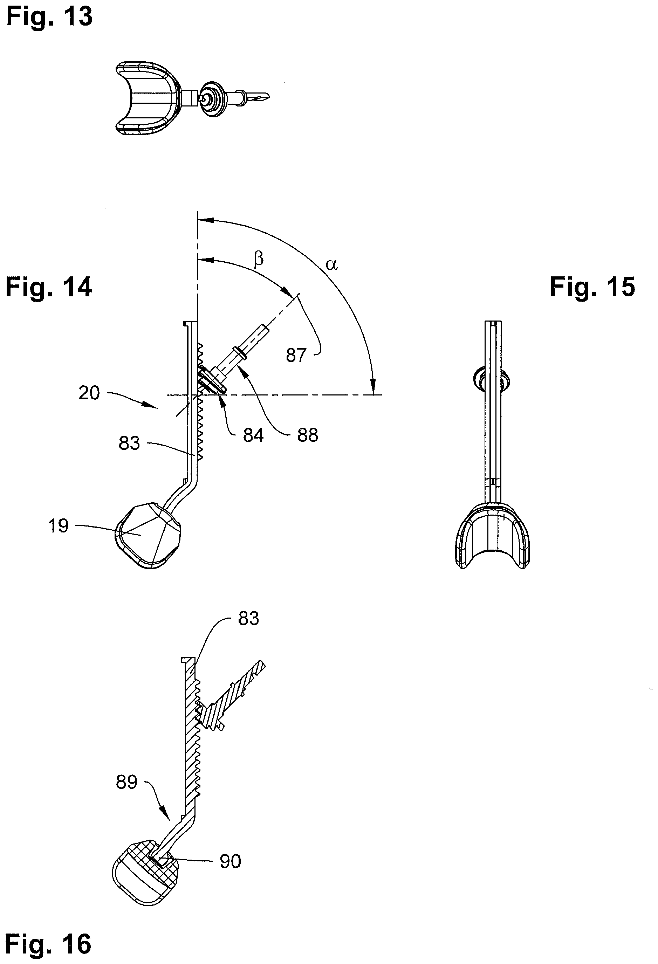

Eine Zusammenschau der **Fig. 13** bis **16** zeigt das Verstellgetriebe 20 der Nasenauflagevorrichtung 18 mit einer Zahnstange 83 und einem Kegelschneckenrad 84 sowie der Nasenauflage 19. Das Verstellgetriebe 20 ist in einem Gehäuse 85 aufgenommen und über das Gehäuse 85 mit der Schwenkachse 17 verbunden. Eine Verstellung der Nasenauflagevorrichtung 18 über das Verstellgetriebe 20 erfolgt durch eine manuelle Betätigung des Bedienelements 62, welches auf eine Betätigungswelle 88 des Kegelschneckenrades 84 aufgesteckt ist. Das Kegelschneckenrad 84 weist einen Öffnungswinkel α von 90° auf, sodass das Kegelschneckenrad 84 in einem Winkel β von 45° relativ zur Zahnstange 83 angeordnet ist. Dies hat zur Folge, dass das Bedienelement 62 des Kegelschneckenrades 84 stets oberhalb einer Transversalebene 86 der Brücke 14 angeordnet ist. Wird die Nasenauflagevorrichtung 18 mit dem Gehäuse 85 an der Schwenkachse 17 der Brücke 14 verstellt, ist eine Längsachse 87 des Kegelschneckenrades 84 dennoch stets um einen Winkel γ > 0° gegenüber der Transversalebene 86 geneigt. Das Bedienelement 62 des Kegelschneckenrades 84 liegt damit immer außerhalb oder am Rand eines Gesichtsfeldes eines Probanden und ist für eine Bedienperson gut erreichbar. Die Lage des Bedienelements 62 stört darüber hinaus auch nicht bei einem Wechsel von hier nicht dargestellten Einsteckgläsern.

Das Kegelschneckenrad 84 ist an die Betätigungswelle 88 einstückig angeformt. Auf die Betätigungswelle 88 ist wiederum das Bedienelement 62 aufgesteckt. Die Zahnstange 83 ist an einem unteren Ende 89 gebogen ausgebildet und weist eine Aufnahmeachse 90 auf. Auf die Aufnahmeachse 90 ist die Nasenauflage 19, die aus einem flexiblen Kunststoffmaterial ausgebildet ist, lediglich aufgesteckt. Die Nasenauflage 19 kann so bei Bedarf leicht ausgewechselt werden.

## Patentansprüche

1. Messbrille (10) zur Bestimmung der subjektiven Refraktion eines Probanden, mit zwei Glashaltevorrichtungen (12, 13) zur Aufnahme von Einsteckgläsern, einer Brücke (14), die die Glashaltevorrichtungen relativ zueinander in einem Abstand verstellbar verbindet, einer Nasenauflagevorrichtung (18), mit einer verschwenkbaren und höhenverstellbaren Nasenauflage (19) und zwei Bügeln (21, 22), die längenveränderbar und höhenverstellbar ausgebildet sind, wobei die Brücke und die Glashaltevorrichtungen zusammen eine Führungseinrichtung (79) zur Führung der bewegbaren Glashaltevorrichtungen in Längsrichtung der Brücke ausbilden, wobei die Brücke ein Spindelgetriebe mit einer Gewindespindel (66) zur Abstandsverstellung der Glashaltevorrichtungen aufweist, wobei an den Glashaltevorrichtungen jeweils zumindest ein Führungsvorsprung (76, 77) ausgebildet ist, der in eine in der Brücke ausgebildeten Führungsnut (75) eingreift, wobei an den Glashaltevorrichtungen jeweils offene Gewindeabschnitte (69) ausgebildet sind, wobei die Gewindespindel mit dem Gewindeabschnitt in Eingriff steht.

2. Messbrille nach Anspruch 1,
**dadurch gekennzeichnet,**
**dass** die Brücke (14) einen Längsschlitz (72) aufweist, der parallele Führungsflächen (73) für die Glashaltevorrichtungen (12, 13) ausbildet, wobei die Führungsnut (75) in zumindest einer der Führungsflächen ausgebildet ist.

3. Messbrille nach Anspruch 2,
**dadurch gekennzeichnet,**
**dass** in Längsrichtung der Brücke (14) eine Durchgangsbohrung (71) zur Aufnahme der Gewindespindel (66) ausgebildet ist, wobei die Durchgangsbohrung an den Längsschlitz (72) anschließt.

4. Messbrille nach einem der vorangehenden Ansprüche,
**dadurch gekennzeichnet,**
**dass** an der Glashaltevorrichtung (12, 13) eine Mehrzahl von stegförmigen Führungsvorsprüngen (76, 77) ausgebildet ist.

5. Messbrille nach einem der vorangehenden Ansprüche,
**dadurch gekennzeichnet,**
**dass** die Brücke (14) aus einem Strangpressprofil (70) ausgebildet ist.

6. Messbrille nach einem der vorangehenden Ansprüche,
**dadurch gekennzeichnet,**
**dass** die Gewindespindel (66) an einem Innenlager (67) radial und an einem Außenlager (68) radial und axial gelagert ist.

7. Messbrille nach Anspruch 6,
**dadurch gekennzeichnet,**
**dass** das Außenlager (68) formschlüssig in die Führungsnut (75) eingreift.

8. Messbrille nach einem der vorangehenden Ansprüche,
**dadurch gekennzeichnet,**
**dass** Bedienelemente (62) zylinderförmig oder kegelstumpfförmig und aus flexiblem Kunststoffmaterial einstückig ausgebildet sind.

9. Messbrille nach einem der vorangehenden Ansprüche,
**dadurch gekennzeichnet,**
**dass** die Brücke (14) aus zwei Brückenabschnitten (15, 16) ausgebildet ist, die mittels einer Schwenkachse (17) fest verbunden sind, wobei an der Schwenkachse die Nasenauflagevorrichtung (18) schwenkbar gelagert ist.

10. Messbrille nach einem der vorangehenden Ansprüche,
**dadurch gekennzeichnet,**
**dass** die Nasenauflagevorrichtung (18) ein Verstellgetriebe (20) zur Höhenverstellung der Nasenauflage aufweist, wobei das Verstellgetriebe eine Zahnstange (83) und ein Kegelschneckenrad (84), die miteinander in Eingriff stehen, aufweist.

11. Messbrille nach einem der vorangehenden Ansprüche,
**dadurch gekennzeichnet,**
**dass** die Glashaltevorrichtung (12, 13) einen drehbaren Ring (50, 51), aufweist, an dem Glasaufnahmen (52, 53) ausgebildet sind, wobei eine Betätigungswelle des Rings eine einstellbare Bremseinrichtung (63) aufweist, wobei die Bremseinrichtung ein exzentrischer Kunststoffring (64) ist.

12. Messbrille nach einem der vorangehenden Ansprüche,
**dadurch gekennzeichnet,**
**dass** die Glashaltevorrichtungen (12, 13) jeweils vier vordere und zwei hintere Glasaufnahmen (52, 53, 54, 55) aufweisen.

13. Messbrille nach einem der vorangehenden Ansprüche,
**dadurch gekennzeichnet,**
**dass** Glasaufnahmen (52, 53, 54, 55) der Glashaltevorrichtungen (12, 13) jeweils zumindest einen Andruckfinger (59) zum Klemmen eines Einsteckglases aufweisen, wobei die jeweiligen Andruckfinger in einer Reihenanordnung an der Glashaltevorrichtung angeordnet und über eine gemeinsame Blattfeder (60) mit einer Andruckkraft beaufschlagbar sind.

14. Messbrille nach einem der vorangehenden Ansprüche,
**dadurch gekennzeichnet,**
**dass** Bügelenden (25, 26) der Bügel (21, 22) jeweils flexibel ausgebildet sind, wobei die Bügelenden jeweils schlaufenförmig ausgebildet sind.

15. Messbrille nach einem der vorangehenden Ansprüche,
**dadurch gekennzeichnet,**
**dass** Bügelenden (25, 26) der Bügel (21, 22) jeweils einstückig sowie aus einem weichen und einem vergleichsweise harten Kunststoffmaterial ausgebildet sind.

16. Messbrille nach einem der vorangehenden Ansprüche,
**dadurch gekennzeichnet,**
**dass** ein ventrales Ende (35, 36) des Bügels (21, 22) gekröpft ausgebildet ist.

17. Messbrille nach einem der vorangehenden Ansprüche,
**dadurch gekennzeichnet,**
**dass** die Messbrille (10) zwei Polarisationsfiltervorrichtungen (11) umfasst.

18. Messbrille nach Anspruch 17,
**dadurch gekennzeichnet,**
**dass** die Polarisationsfiltervorrichtungen (11), die jeweils an den Glashaltevorrichtungen (12, 13) mittels einer Rastverbindung (43) lösbar befestigbar sind.

19. Messbrille nach Anspruch 18,
**dadurch gekennzeichnet,**
**dass** an einem Befestigungsende eines Halters (44, 45) der Polarisationsfiltervorrichtung (11) eine einstückige Federklemme (46) ausgebildet ist, die einen an der Glashaltevorrichtung (12, 13) angeformten Steg (23, 24) hintergreift, wobei an der Federklemme ein Betätigungshebel (48) angeformt ist, mittels dem die Federklemme öffenbar ist.

20. Messbrille nach einem der Ansprüche 17 bis 19,
**dadurch gekennzeichnet,**
**dass** die Polarisationsfiltervorrichtungen (11) einen zirkularen oder einen linearen Polarisationsfilter aufweisen, wobei die Polarisationsfiltervorrichtungen mit dem zirkularen oder linearen Polarisationsfilter (91) jeweils eine Schwenkeinrichtung (93) zum Ein- und Ausschwenken des zirkularen oder linearen Polarisationsfilters vor die Glashaltevorrichtung (12, 13) aufweisen, wobei die Polarisationsfiltervorrichtungen mit dem linearen Polarisationsfilter jeweils eine Dreheinrichtung (92) zum Drehen des linearen Polarisationsfilters vor der Glashaltevorrichtung aufweisen.

## Claims

1. A trial frame (10) for determining the subjective refraction of a subject, comprising two lens holder devices (12, 13) for receiving insert lenses, a bridge (14), which connects the lens holder devices in such a manner that their distance relative to each other is adjustable, a nose rest device (18) having a pivotable and height-adjustable nose rest (19) and two temples (21, 22), which are adjustable in length and height, the bridge and the lens holder devices together forming a guide means (79) for guiding the mobile lens holder devices in the longitudinal direction of the bridge, the bridge having a spindle gear comprising a threaded spindle (66) for adjusting the distance of the lens holder devices,
wherein
at least one guide protrusion (76, 77) is formed on each of the lens holder devices, said guide protrusion engaging into a guide groove (75) formed in the bridge, open threaded sections (69) being formed on each of the lens holder devices, the threaded spindle being engaged with the threaded section.

2. The trial frame according to claim 1,
**characterized in that**
the bridge (14) has a longitudinal slot (72) which forms parallel guide surfaces (73) for the lens holder devices (12, 13), the guide groove (75) being formed in at least one of the guide surfaces.

3. The trial frame according to claim 2,
**characterized in that**
a passage bore (71) for receiving the threaded spindle (66) is formed in the longitudinal direction of the bridge (14), the passage bore being adjacent to the longitudinal slot (72).

4. The trial frame according to any one of the preceding claims,
**characterized in that**
a plurality of crosspiece-shaped guide protrusions (76, 77) is formed on the lens holder device (12, 13).

5. The trial frame according to any one of the preceding claims,
**characterized in that**
the bridge (14) is formed by an extruded profile (70).

6. The trial frame according to any one of the preceding claims,
**characterized in that**
the threaded spindle (66) is mounted radially on an inner bearing (67) and mounted radially and axially on an outer bearing (68).

7. The trial frame according to claim 6,
**characterized in that**
the outer bearing (68) engages into the guide groove (75) in a form-fitting manner.

8. The trial frame according to any one of the preceding claims,
**characterized in that**
operating elements (62) are cylindrical or frustoconical in shape and are made in one piece of a flexible plastic material.

9. The trial frame according to any one of the preceding claims,
**characterized in that**
the bridge (14) is formed by two bridge sections (15, 16), which are firmly connected by means of a pivot axis (17), the nose rest device (18) being pivotably mounted on the pivot axis.

10. The trial frame according to any one of the preceding claims,
**characterized in that**
the nose rest device (18) has an adjustment gear (20) for adjusting the height of the nose rest, the adjustment gear having a toothed rack (83) and a bevel worm wheel (84), which are engaged with each other.

11. The trial frame according to any one of the preceding claims,
**characterized in that**
the lens holder device (12, 13) has a rotatable ring (50, 51), on which lens seats (52, 53) are formed, an actuating shaft of the ring having an adjustable braking means (63), the braking means being an eccentric plastic ring (64).

12. The trial frame according to any one of the preceding claims,
**characterized in that**
the lens holder devices (12, 13) each have four front and two rear lens seats (52, 53, 54, 55).

13. The trial frame according to any one of the preceding claims,
**characterized in that**
each lens seat (52, 53, 54, 55) of the lens holder devices (12, 13) has at least one pressure finger (59) for clamping an insert lens, the respective pressure fingers being arranged in a row on the lens holder device and being subjectable to a contact pressure force via a shared leaf spring (60).

14. The trial frame according to any one of the preceding claims,
**characterized in that**
each temple end (25, 26) of the temples (21, 22) is flexible, each temple end being loop-shaped.

15. The trial frame according to any one of the preceding claims,
**characterized in that**
each temple end (25, 26) of the temples (21, 22) is formed in one piece and is made of a soft and of a comparatively hard plastic material.

16. The trial frame according to any one of the preceding claims,
**characterized in that**
a ventral end (35, 36) of the temple (21, 22) is cranked.

17. The trial frame according to any one of the preceding claims,
**characterized in that**
the trial frame (10) comprises two polarization filter devices (11).

18. The trial frame according to claim 17,
**characterized in that**
the polarization filter devices (11) are each removably attachable to the lens holder devices (12, 13) by means of a locking connection (43).

19. The trial frame according to claim 18,
**characterized in that**
a one-piece spring clamp (46) is formed on an attachment end of a holder (44, 45) of the polarization filter device (11), said spring clamp engaging behind a crosspiece (23, 24) molded to the lens holder device (12, 13), an actuating lever (48) being molded to the spring clamp, by means of which the spring clamp can be opened.

20. The trial frame according to any one of claims 17 to 19,
**characterized in that**
the polarization filter devices (11) have a circular or a linear polarization filter, the polarization filter devices having the circular or linear polarization filter (91) each having a pivot means (93) for pivoting the circular or linear polarization filter in and out in front of the lens holder device (12, 13), the polarization filter devices having the linear polarization filter each having a rotating means (92) for rotating the linear polarization filter in front of the lens holder device.

## Revendications

1. Monture de lunettes d'essai (10) pour déterminer la réfraction subjective d'un participant à un examen, ayant deux dispositifs (12, 13) de support de verres pour recevoir des verres de lunettes embrochables, un pont (14) qui relie les dispositifs de support de verres de façon à avoir une distance ajustable entre eux, un dispositif (18) d'appui de nez ayant un appui de nez (19) pivotable et réglable en hauteur et deux branches (21, 22) qui sont variables en longueur et réglables en hauteur, le pont et les dispositifs de support de verres formant une unité de guidage (79) pour guider les dispositifs de support de verres mobiles dans le sens longitudinal du pont, le pont présentant un engrenage de broche ayant une broche filetée (66) pour ajuster la distance des dispositifs de support de verres,
dans lequel
au moins une partie saillante de guidage (76, 77) est formée à chacun des dispositifs de support de verres, ladite partie saillante de guidage s'engageant dans une rainure de guidage (75) formée dans le pont, des sections filetées (69) étant formées à chacun des dispositifs de support de verres, la broche filetée étant engagée avec la section filetée.

2. Monture de lunettes d'essai selon la revendication 1,
**caractérisée en ce que**
le pont (14) présente une fente longitudinale (72) qui forme des surfaces de guidage (73) parallèles pour les dispositifs (12, 13) de support de verres, la rainure de guidage (75) étant formée dans au moins une des surfaces de guidage.

3. Monture de lunettes d'essai selon la revendication 2,
**caractérisée en ce qu'**
un perçage traversant (71) pour recevoir la broche filetée (66) est formé dans le sens longitudinal du pont (14), le perçage traversant bordant la fente longitudinale (72).

4. Monture de lunettes d'essai selon l'une quelconque des revendications précédentes,
**caractérisée en ce qu'**
une pluralité de parties saillantes de guidage (76, 77) en forme de passerelle est formée au dispositif (12, 13) de support de verres.

5. Monture de lunettes d'essai selon l'une quelconque des revendications précédentes,
**caractérisée en ce que**
le pont (14) est formé par un profil extrudé (70).

6. Monture de lunettes d'essai selon l'une quelconque des revendications précédentes,
**caractérisée en ce que**
la broche filetée (66) est montée radialement à un palier intérieur (67) et montée radialement et axialement à un palier extérieur (68).

7. Monture de lunettes d'essai selon la revendication 6,
**caractérisée en ce que**
le palier extérieur (68) s'engage dans la rainure de guidage (75) à engagement positif.

8. Monture de lunettes d'essai selon l'une quelconque des revendications précédentes,
**caractérisée en ce que**
des éléments de commande (62) sont cylindriques ou tronconiques et sont réalisés intégralement d'une matière plastique flexible.

9. Monture de lunettes d'essai selon l'une quelconque des revendications précédentes,
**caractérisée en ce que**
le pont (14) est formé par deux sections de pont (15, 16) qui sont reliées de manière à être solidaire au moyen d'un axe de pivotement (17), le dispositif (18) d'appui de nez étant monté de manière pivotable à l'axe de pivotement.

10. Monture de lunettes d'essai selon l'une quelconque des revendications précédentes,
**caractérisée en ce que**
le dispositif (18) d'appui de nez présente un mécanisme de réglage (20) pour ajuster la hauteur de l'appui de nez, le mécanisme de réglage présentant un arbre denté (83) et une roue conique hélicoïdale (84) qui sont engagés l'un avec l'autre.

11. Monture de lunettes d'essai selon l'une quelconque des revendications précédentes,
**caractérisée en ce que**
le dispositif (12, 13) de support de verres présente un anneau rotatif (50, 51) sur lequel des prises de verres (52, 53) sont formées, un arbre d'actionnement de l'anneau présentant une unité de freinage (63) ajustable, l'unité de freinage étant un anneau plastique (64) excentrique.

12. Monture de lunettes d'essai selon l'une quelconque des revendications précédentes,
**caractérisée en ce que**
les dispositifs (12, 13) de support de verres chacun présentent quatre prises de verres (52, 53) antérieures et deux prises de verres (54, 55) arrières.

13. Monture de lunettes d'essai selon l'une quelconque des revendications précédentes,
**caractérisée en ce que**
chaque prise de verre (52, 53, 54, 55) du dispositif (12, 13) de support de verres présente au moins un doigt de pression (59) pour pincer un verre de lunettes embrocha blé, les doigts de pression respectifs étant disposés en série au dispositif de support de verres et une force de pression pouvant agir sur lesdits doigts de pression à travers un ressort à lames (60) commun.

14. Monture de lunettes d'essai selon l'une quelconque des revendications précédentes,
**caractérisée en ce que**
chaque extrémité de branche (25, 26) des branches (21, 22) est réalisée à être flexible, chaque extrémité de branche étant réalisée en forme de boucle.

15. Monture de lunettes d'essai selon l'une quelconque des revendications précédentes,
**caractérisée en ce que**
chaque extrémité de branche (25, 26) des branches (21, 22) est réalisée intégralement ainsi que d'une matière plastique molle et d'une matière plastique comparativement dure.

16. Monture de lunettes d'essai selon l'une quelconque des revendications précédentes,
**caractérisée en ce qu'**
une extrémité (35, 36) ventrale de la branche (21, 22) est réalisée à être coudée.

17. Monture de lunettes d'essai selon l'une quelconque des revendications précédentes,
**caractérisée en ce que**
la monture de lunettes d'essai (10) comporte deux dispositifs (11) de filtre de polarisation.

18. Monture de lunettes d'essai selon la revendication 17,
**caractérisée en ce que**
les dispositifs (11) de filtre de polarisation chacun sont fixable aux dispositifs (12, 13) de support de verres de manière détachable au moyen d'une liaison par verrouillage (43).

19. Monture de lunettes d'essai selon la revendication 18,
**caractérisée en ce qu'**
une borne à ressort (46) d'une seule pièce est formée à une extrémité de fixation d'un support (44, 45) du dispositif (11) de filtre de polarisation, la borne à ressort s'engageant derrière une passerelle (23, 24) moulée au dispositif (12, 13) de support de verres, un levier (48) d'actionnement étant moulé à la borne à ressort, la borne à ressort pouvant être ouverte au moyen dudit levier d'actionnement.

20. Monture de lunettes d'essai selon l'une quelconque des revendications 17 à 19,
**caractérisée en ce que**
les dispositifs (11) de filtre de polarisation présentent un filtre de polarisation circulaire ou linéaire, les dispositifs de filtre de polarisation ayant le filtre (91) de polarisation circulaire ou linéaire chacun présentant une unité (93) de pivotement pour pivoter le filtre de polarisation circulaire ou linéaire vers l'intérieur et vers l'extérieur devant le dispositif (12, 13) de support de verres, les dispositifs de filtre de polarisation ayant le filtre de polarisation linéaire chacun présentant une unité (92) tournante pour tourner le filtre de polarisation linéaire devant le dispositif de support de verres.
